# EUROPEAN PATENT APPLICATION

(11) **EP 4 659 716 A2**
(43) Date of publication of application: **10.12.2025**
(21) Application number: 25210894.9
(22) Date of filing: 07.04.2021
(51) Int. Cl.: A61F 2/90

(54) **BIO-ALLOY BRAIDED SELF-EXPANDING BIODEGRADABLE STENT**

(30) Priority: 07.04.2020 US 202063006565 P
(62) Divisional of application: 21723470.7
(71) Applicant: Zorion Medical, Inc., Zionsville, IN 46077 (US)
(72) Inventor: PAQUIN, Mark, Indianapolis, 46202 (US); BROECKER, David, Zionsville, 46077 (US)
(74) Representative: Barker Brettell LLP

(57) **Abstract**

An implantable device, comprising: a tube comprising a plurality of biodegradable biometallic wires braided together, the tube being coated with a flexible conformal biodegradable polymer in an expanded state such that, upon compression and release of compression, the flexible conformal biodegradable polymer-coated tube self-expands back to the expanded state, wherein the flexible conformal biodegradable polymer binds the braided wires of the tube together at a plurality of wire crossover points to inhibit sliding movement between the wires, and wherein the flexible conformal biodegradable polymer comprises a plasticizer.

## Description

### CROSS-REFERENCE TO RELATED APPLICATIONS

The present application is based on and claims priority from U.S. Patent Application Ser. No. 63/006,565, filed on April 7, 2020, the entire disclosure of which is incorporated herein by reference.

### BACKGROUND

Current bioabsorbable stents, typically made of varying amounts of bioplastics and biometals, have significant performance limitations based on the underlying properties of the biomaterials used to make the stent.

Both bioplastics and biometals lack radiopacity, making delivery and tracking of the device difficult, particularly in clinical situations involving precise vessel sizing and implant placement. In addition, bioplastic scaffolds lack inherent strength and flexibility, particular in comparison to non-absorbable materials.

To compensate for certain properties such as lack of strength, stents made from bioplastics generally require more material (i.e., higher mass, greater strut thicknesses), resulting in longer absorption times (i.e., on the order of years rather than months) following implantation.

Biometals have mechanical properties that are more similar to those of non-absorbable metals; however, to maintain these properties in the implantable device, a limited set of designs and fabrication techniques are available, often resulting in suboptimal performance (e.g., high failure rates due to fracture and/or too rapid degradation) across a narrow set of performance conditions (e.g. vessel diameters) or delivery systems (e.g. balloon-expandable) for deployment.

### SUMMARY

Accordingly, the present disclosure is directed to improved bioabsorbable devices such as stents which address one or more of the problems identified above, including suboptimal performance and limited applications, either due to poor visibility, stent design, or the biomaterial from which these bioabsorbable stents are made and/or a combination of thereof, and specifically addresses issues involving precise vessel sizing and implant placement of bioabsorbable stents. Magnesium stents, and more particularly, self-expanding, braided wire-based magnesium stents, engineered for more rapid absorption times (e.g., over a period of months rather than years) having enhanced visibility, address one or more limitations associated with current bioabsorbable stents described above and in the prior art.

Disclosed herein are various embodiments of a hybrid self-expanding biodegradable stent (HSEBS). For purposes of the present disclosure, the term "hybrid" generally refers to the incorporation of radiopaque (RO) metallic wires (e.g. single, composite, and/or multi-wire strands) that enhance the visibility (e.g. using radiography or other imaging modalities) of the device for implantation. The term "self-expanding" generally refers to the ability of the device to recover a significant portion of the as-made diameter upon delivery inside a lumen. The term "biodegradable stent" generally refers to the ability of the device to safely absorb following implantation based on the predominance of biometal wire components (e.g. single, composite, multi-wire strand) used to make the device. Finally, the term "biometal" and "biometallic" refers to metals that are biocompatible when implanted into a living subject such as a human and which may be biodegradable. The HSEBS may be produced from a braided tube including biodegradable biometallic wires with or without permanently coated RO wires interlaced clockwise and counterclockwise around a mandrel. The addition of radiopaque wires to the HSEBS makes it possible to visualize the entire length of the stent from end-to-end and track the stent during implantation and other procedures.

In various embodiments the biodegradable biometallic wire may be produced from a magnesium-alloy (MA), where the majority of the wire (e.g., at least 80%) may be magnesium. In certain embodiments, the RO wire may be produced from non-degradable metals. In certain embodiments the MA may be made of medical-grade materials and may be free of rare earth elements.

In certain embodiments the MA may be alloyed magnesium (e.g., greater than 90% w/w Mg) which may also include zinc, calcium, and manganese to produce an alloy that is strong and ductile, and free of rare earth elements, making the alloy suitable for implantation inside various structures including blood vessels, such as arteries and veins.

In particular embodiments the MA may be chemically composed of zinc, zirconium, and rare earth elements with at least 80% w/w of magnesium, where the MA may be biocompatible and have high tensile strength, yield strength, and percent elongation. In some embodiments the biodegradable metal may also include iron and zinc.

In some embodiments, the RO wire may be made from metals having lower elastic modulus and high yield stress for large elastic strains that play both a radiopacity and mechanical role in the braided structure. Elastic modulus is a key factor of the springiness of a metal and the ability of a metal/alloy to recover from a compressed state, and is essentially, a fixed material property that is inherent to an alloy/material system. In some alloys, the elastic modulus can be changed and increased slightly by further processing of the metal/alloy. For example, in terms of elasticity (lowest to highest), magnesium has a low elastic modulus compared to other braidable metals/alloys at 5 megapounds per square inch (Mpsi), while nitinol or nickel-titanium's (~8-12 Mpsi), titanium's (10-14 Mpsi), stainless, platinum, and tantalum's (20-25 Mspi), and cobalt-chromium's (25-30 Mpsi) are significantly higher.

The RO wire may be produced from biocompatible metals, including gold, platinum, and tantalum, as these metals have a higher density than the material of the stent, allowing the stent to be easily seen radiographically. In certain embodiments the diameter of the RO wire may be reduced by between 25% and 40% compared to the diameter of the magnesium alloy wire, which can help to compensate for the mechanical properties of the RO wire and so as not to distort the HSEBS which incorporates the RO wire.

In particular embodiments, the RO wire may be a composite material, which may be made from a MA tube or a shape memory alloy, such as nickel-titanium (or nitinol), and which may contain a core material to enhance visibility. In some embodiments, particularly the latter shape memory alloy, the composite wire may provide both visibility and mechanical support to the braided structure. In some embodiments, the biodegradable tubed MA composite material may contain a core of radiopacifying powdered material, which provides radiodensity to a typically radiolucent material.

In other embodiments, the radiopaque element may include a strand of wires wrapped around a RO core wire that may play both a radiopacity and mechanical role in the braided structure.

In some embodiments, the plurality of wires that make up the braided tube may be wound and woven in a one-over, one-under (1x1) pattern in a clockwise and counterclockwise manner around a single mandrel. In other embodiments, the wires may be wound and woven in a 2x2 (two-over, two-under) braided pattern. In general, braiding of wires means having wires wrapped in a tube shape (e.g., around a mandrel) running in clockwise and counterclockwise directions which are woven together, for example running under and over one another as they cross.

In certain embodiments, the braided tube may be initially formed in its expanded shape, such that the braided tube will recover or re-expand back to an expanded state following compression, and in one particular embodiment a preferred number of wires is twenty-four (24). In various embodiments, the angles of the braided wires is not less than 30° relative to the mandrel's longitudinal axis at the point where the wires intersect.

In particular embodiments, the ratio and/or arrangement of permanently coated non-degradable metallic wires to biodegradable biometallic wires may be no more than 1:1 of the total number of wires needed to produce the braided tube. In an embodiment for a 24-wire braided configuration, the ratio may not exceed 1:11 as most (>90%) of the braided construct is to be absorbable unless composite wires are used.

In some embodiments, the RO wire may be coated with a permanent (i.e. non-biodegradable) polymer coating to prevent the dissimilar metals from coming into contact, thereby inhibiting or preventing galvanic corrosion. In various embodiments, the polymer may be made from a dielectric, insulating material that can withstand very high temperatures.

In particular embodiments, the tube may be cut into smaller segments (or "braided stents") of varying lengths. In certain embodiments, the braided tube may be cut into braided stents at a point where the wires complete at least one full revolution (360°) along the long axis (e.g., on the mandrel) to reduce the probability of the wire ends from unraveling. In some embodiments, the hybrid tube may undergo a first cut to separate a shorter braided segment from the hybrid tube and a finishing-cut to produce an uniform end length to the stent to reduce the risk of wire deformation and improve stent symmetry and uniformity.

In some embodiments, the end wires may be joined together to close the end of the device using joining cuffs including both absorbable and non-absorbable elements. In other embodiments, the end of the stent may be closed during the braiding process by looping a wire back into the pattern for the braided tube.

In certain embodiments, the braided stent may be self-expanding, where the self-expanding stent may recover from compression to a diameter of at least 50% of its as-made diameter (i.e. the diameter of the pre-compression shape) after being compressed, constrained, and released from a delivery system, such as a catheter.

In various embodiments, the stent may be coated with a biodegradable polymer. In some embodiments, the coating may be applied by spraying layers and/or dip-coating layers onto the braided stent, creating a conformable and flexible coating, where the biodegradable polymer coating may be utilized to modulate the Mg-based substrate's bio-absorption profile when implanted into tissue.

The conformal and flexible coating uniformly binds the wires at crossover points to prevent the wires from sliding relative to one another while adhering to the surface of the stent. The applied coating may, in certain embodiments, reduce or prevent deformation of the wire as well as allow the braided stent to achieve uniform expansion once unconstrained.

Conformal and flexible coatings of the type described herein also improve the mechanical properties of the stent, particularly the ability of the stent to recover or re-expand back to the desired diameter and shape and, importantly, reduce or prevent the distortion of the stent ends. This may be achieved by the combination of the elastic properties of the stent and the conformal, flexible coating, the latter being used to control both the bio-absorption profile and to improve the recovery or re-expansion capability of the stent. In particular embodiments, the conformal biodegradable polymer may include a plasticizer. In some embodiments, the conforming properties of the polymer may be optionally modified with the use of various biocompatible plasticizers. In some embodiments, the polymer with plasticizer helps in the elastic recovery of the stent during deployment.

In various embodiments the conformal, flexible biodegradable polymer coating (with or without the addition of a plasticizer) may be applied evenly to the entire stent resulting in a coating with approximately the same thickness of 10 micrometers covering the whole stent. In some embodiments, the coating thickness may be less than and/or exceed 10 micrometers to speed-up corrosion or extend the biodegradable metal's mechanical properties' longevity.

In particular embodiments, the coating may be applied in layers to the stent to achieve a coating thickness of approximately 10 micrometers at the middle of the stent and a higher thickness, up to 30 micrometers but preferably about 20 micrometers, at each of the opposite ends of the stent starting from each edge and covering approximately 5% to 50% of the stent length and preferably encapsulating about 10% to 20% of the stent length. In some embodiments, discrete bands and/or rings may be applied to encapsulate and/or suspend the open-end wire ends of the braided stent.

Accordingly, one embodiment provides an implantable device, including: a tube including a plurality of biodegradable biometallic wires braided together, the tube being coated with a flexible conformal biodegradable polymer in an expanded state such that, upon compression and release of compression, the flexible conformal biodegradable polymer-coated tube self-expands back to the expanded state.

### BRIEF DESCRIPTION OF THE DRAWINGS

Various objects, features, and advantages of the disclosed subject matter can be more fully appreciated with reference to the following detailed description of the disclosed subject matter when considered in connection with the following drawings, in which like reference numerals identify like elements.

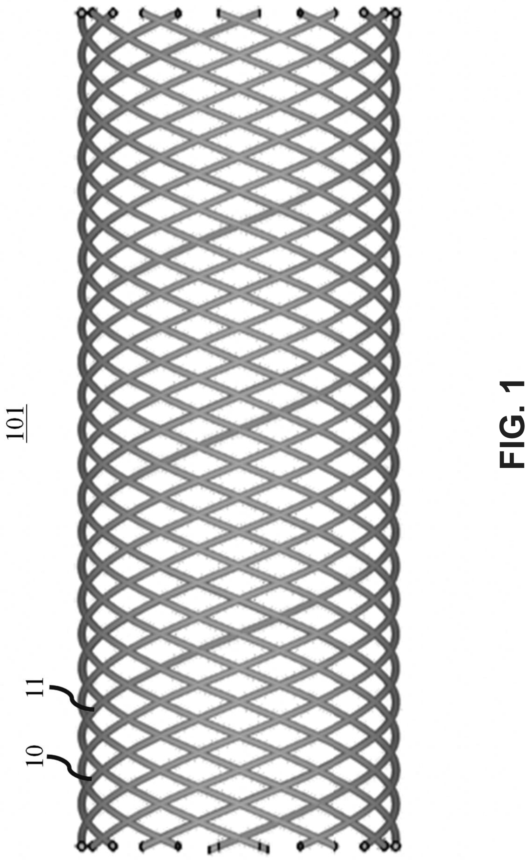

FIG. 1 shows a HSEBS produced from a braided tube including biodegradable biometallic wires 10 and RO wires 11 interlaced in a clockwise and counterclockwise manner.

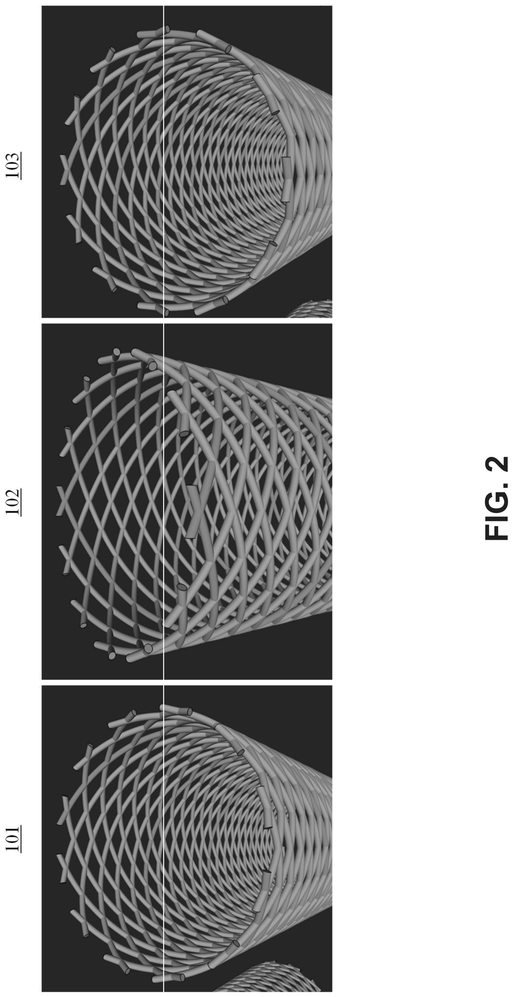

FIG. 2 shows different configurations in which the RO wires can be disposed. For example, in stent 101 (left panel) there are two RO wires which are disposed opposite (or 180° apart) from one another when viewed in cross-section. In this particular 2-RO-wire configuration, the RO wires provide radiopacity to the braided structure. In stent 102 (middle panel) there are 4 RO wires, 2 of which wrap in the clockwise direction and 2 of which wrap in the counterclockwise direction, such that the points of intersection of the wires are disposed opposite (or 180° apart) from one another when viewed in cross-section, as in the 2-RO-wire configurations. In this case, the RO wires provide a radiopacity role and may provide a mechanical role. Stent 103 (right panel) on the other hand is a 4-RO-wire configuration in which all of the wires are wrapped in the same direction and are placed 90° apart (when viewed in cross-section) for optimal visibility and for added design stability, particularly for thinner wire diameter braided stents.

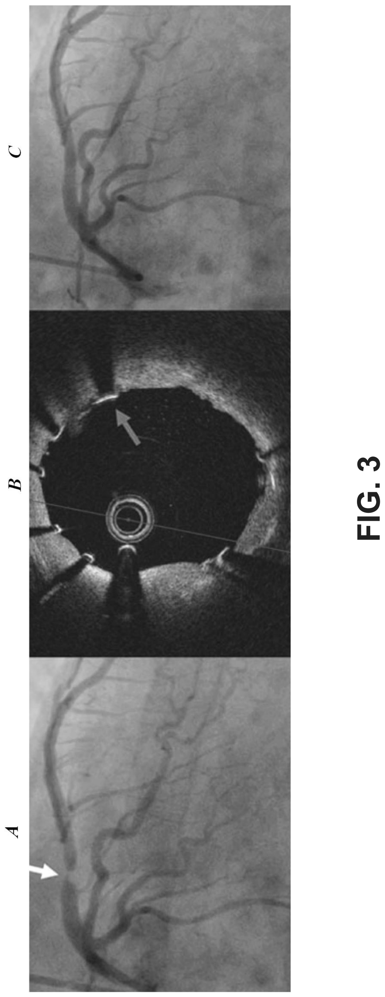

FIG. 3 shows several images which demonstrate the lack of visibility afforded by magnesium and magnesium alloys. Image A shows a stenotic lesion (white arrow). Image B provides an intravascular image of an implanted magnesium stent strut (green arrow). Magnesium and magnesium alloy stents are visible under endovascular imaging such as intravascular ultrasound but remain undetectable under fluoroscopic imaging (image C).

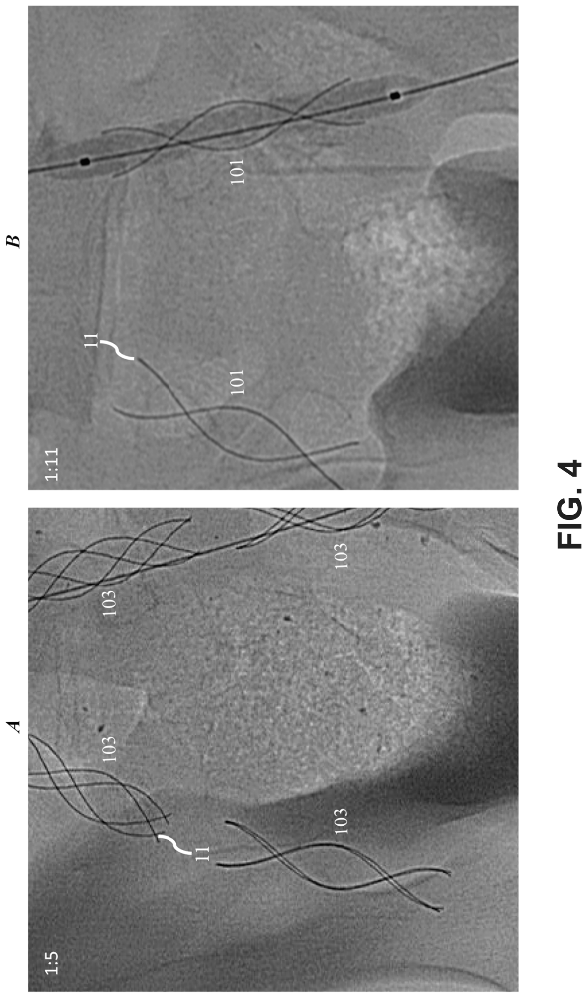

FIG. 4 provides cinegraphic examples of implanted hybrid braided self-expanding biodegradable stent (HSEBS) in the arteries of domestic swine. Image A shows stents 103 having 20 magnesium alloy (MA) wires and 4 RO wires. The RO-wire to MA wire disposition if 1:5. Image B shows stent 101 with a RO to MA wire ratio of 1:11 where the wires are disposed opposite (or 180° apart) from one another when viewed in cross-section.

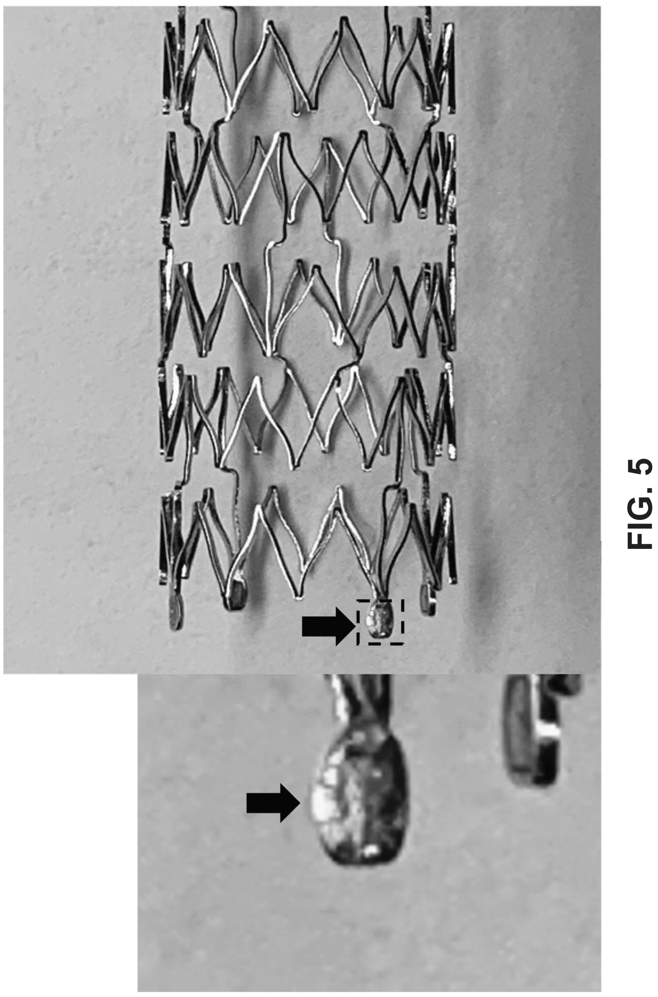

FIG. 5 shows a non-degradable laser-cut metallic stent with discrete markers added to the ends of the stent to enhance the visibility of the stent, particularly at its extremities (see arrows in main image and in the inset, where the inset corresponds to the dashed box in the main image). These markers may be made from gold, platinum, or tantalum. The markers generally have a higher density than the material of the stent, allowing the ends (or other extremities) of the stent to be readily seen angiographically.

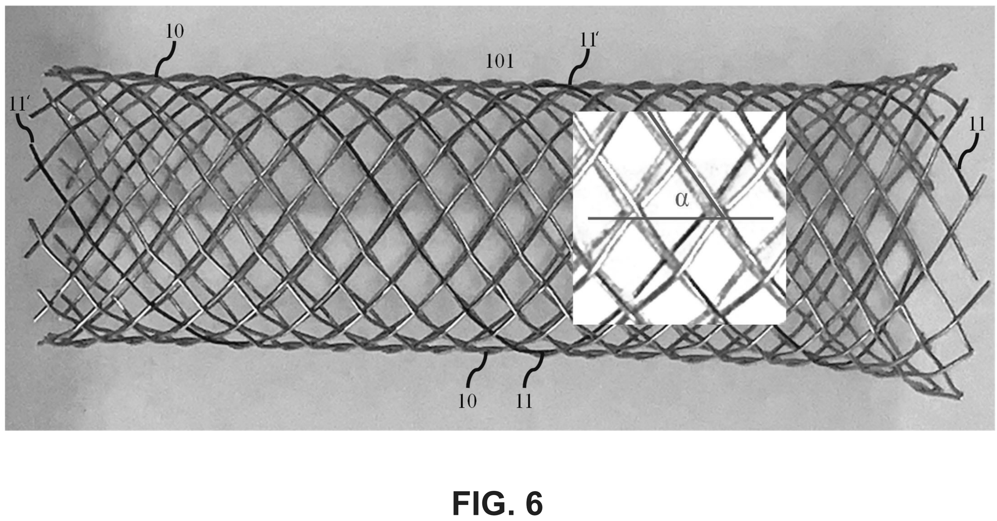

FIG. 6 is an image of a 24-wire HSEBS disposed in a 1:11 RO to MA wire configuration where RO wires 11 are disposed opposite (or 180° apart) from one another when viewed in cross-section in the two-RO-wire configurations. RO wire 11' denotes the RO wire spanning the length of the stent. Also shown is the angle α at which the RO and MA wires are wound relative to the longitudinal axis of the braided tube.

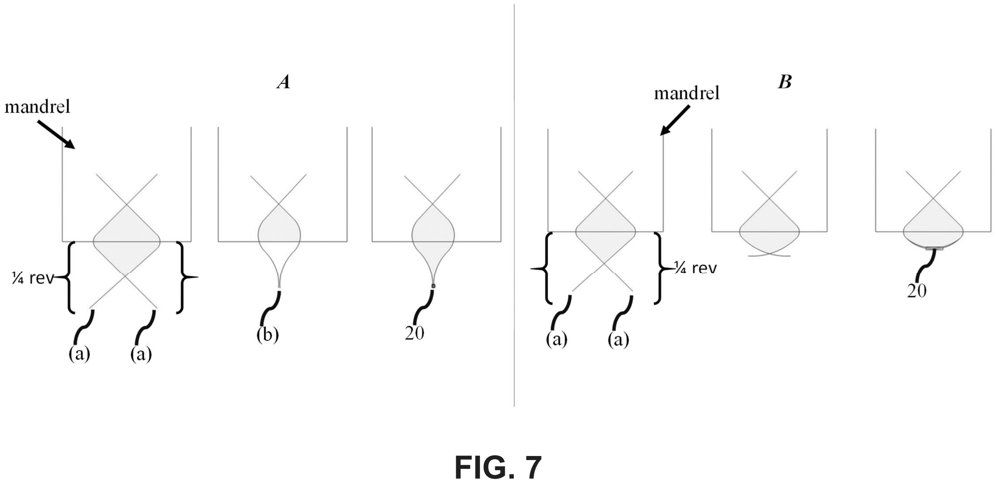

FIG. 7 is a depiction of how the hybrid tube in panel A undergoes a first cut (a) to produce a shorter braided segment from the longer hybrid tube followed by a finishing-cut (b) to produce an approximately uniform end length to the stent to reduce the risk of wire deformation and improve stent symmetry and uniformity, i.e., produce wire ends having substantially the same length as one another. Panel A of FIG. 7 also shows how the hybrid braided tube may undergo the same cutting process of a first rough cut followed by a second cut to trim the wire ends where the wires complete an additional one-quarter revolution along the mandrel's long-axis (a). Then, pairs of wire-ends may be separated and aligned parallel to one another and secured with a polymer cuff 20. Once each pair of end-wires (panel A) is connected (e.g., using cuffs), the wire-ends protruding from the cuffed ends may be trimmed to form a closed-end stent. Alternatively, the pairs of wire-ends may be bent to form a loop (FIG. 7, panel B) and aligned parallel to one another and secured with a polymer cuff 20 by tucking the wires into the cuff.

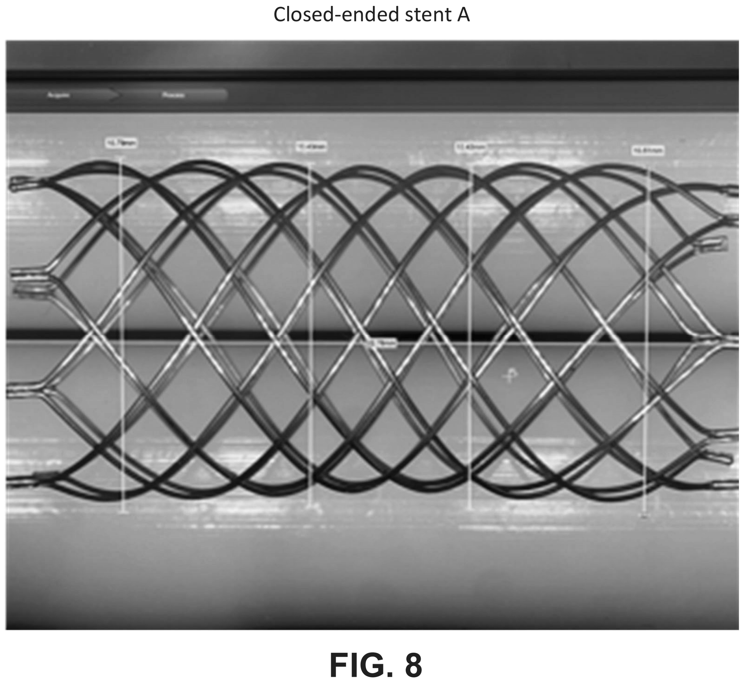

FIG. 8 presents a closed-ended stent A in which pairs of wire-ends may be separated and aligned parallel to one another and secured with a polymer cuff. The stent is produced using a 2x2 braided pattern.

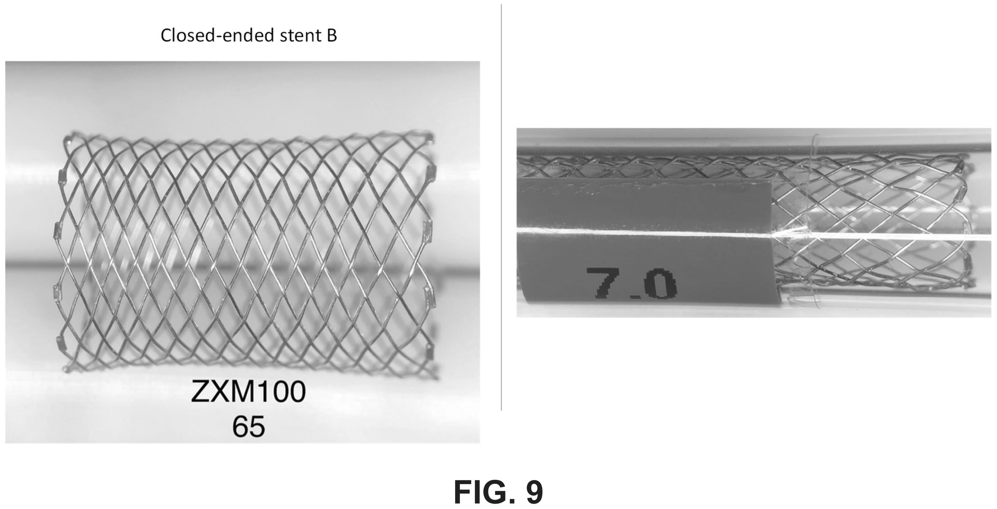

FIG. 9 presents a closed-ended B (left image) where pairs of wire-ends are bent to form a loop and aligned parallel to one another and approximately perpendicular to the longitudinal axis of the stent and secured with a polymer cuff by tucking the wires into the cuff. The stent in this case is produced from MA wire containing zinc, calcium, and manganese (ZXM) in a 1x1 braided pattern with a 65° braid angle along the x-axis/relative to the longitudinal axis. The right image in FIG. 9 is of the ZXM braided closed-ended stent after it was compressed to a loading diameter and loaded into a 7 mm glass tube to simulate the deployment of the stent into a blood vessel.

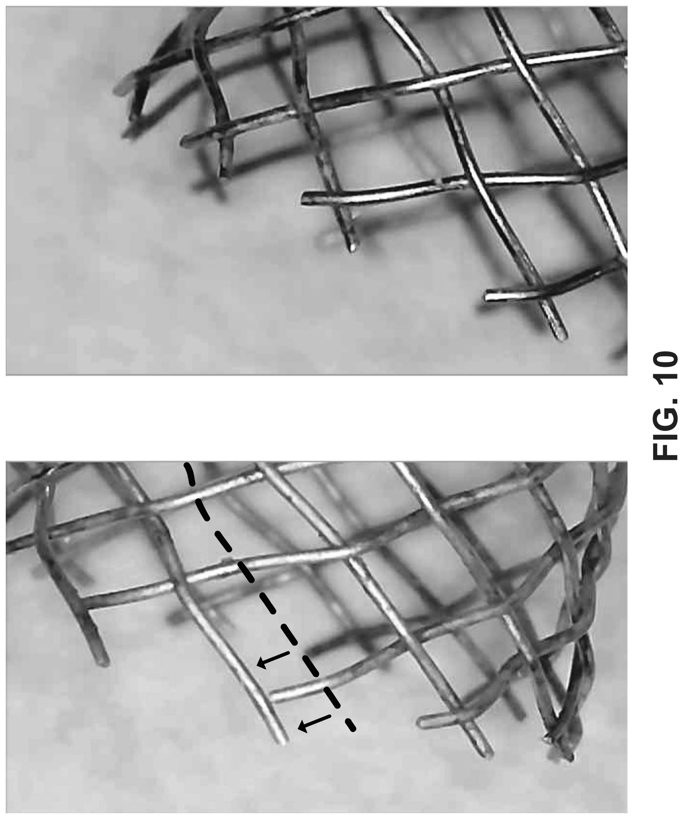

FIG. 10 shows braided stents, both having a conformal biodegradable polymer (CBP) coating. The left image is of a stent coated with a CBP coating without a plasticizer and the right image is of a stent coated with a CBP coating with a plasticizer. The CBP coating with or without a plasticizer is used to bind the wires at the crossover points to prevent them from sliding relative to one another. However, only the CBP coating that includes plasticizer helps prevent deformation of the wire ends and helps prevent the wires from detaching at the crossover points, particularly in the open-ended configured stents, where the wire ends of the stents are at higher risk of deformation and polymer delamination during the stent's cycling process (e.g., crimping, loading, and expansion). The left image in FIG. 10 includes a dashed line which shows the original location of a wire which has detached at several crossover points and as a result has slid relative to the other wires (indicated by the arrows). On the other hand, the wires of the stent in the right image in FIG. 10 have not detached at crossover points.

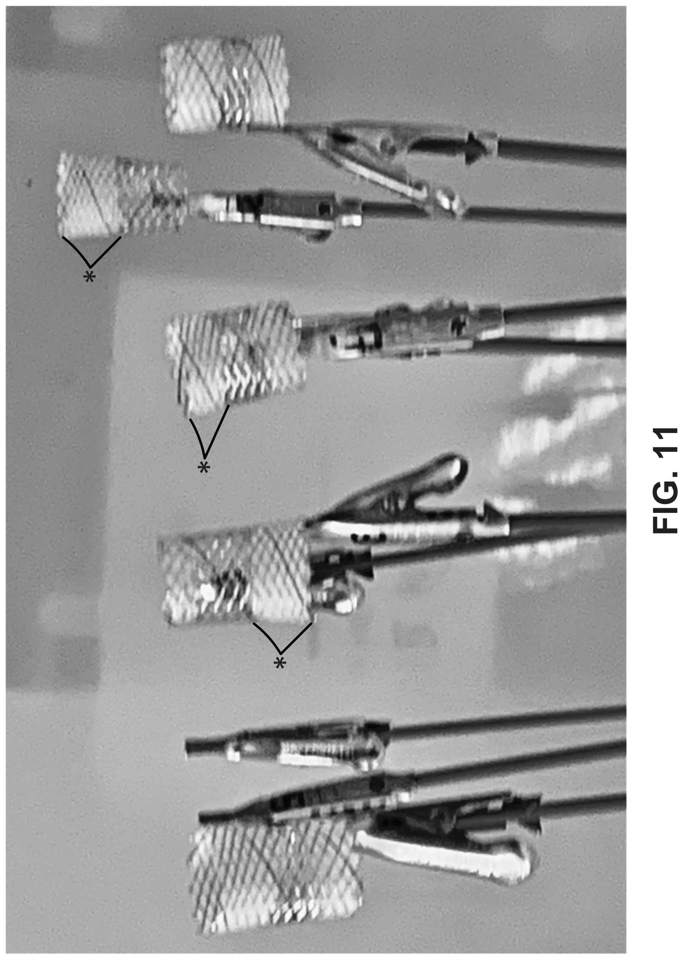

FIG. 11 shows the CBP coating can be applied in a thicker layer at the edges or ends of each stent segment, with several examples being indicated using lines and an asterisk (*), in order to form polymer rings to thoroughly encapsulate the open-ended wire ends. Producing the polymer rings at the ends of the stent segments can be achieved by a number of procedures including dipping, spraying, and/or painting the rings onto the edges of the stents one or more times in the polymer solution and/or by applying the polymer to the stent's edges using a higher concentration of polymer solution (e.g., 2x).

### DETAILED DESCRIPTION

In accordance with some embodiments of the disclosed subject matter, mechanisms (which can include apparatus, systems, methods, and media) for improved bioabsorbable devices such as stents provided along with the procedures for making and using such devices.

This disclosure presents embodiments for a new hybrid bioabsorbable self-expanding, wire-based braided stent to address, among other issues, the durability problems associated with permanent stents and stent-like devices as well as to address the lack of mechanical strength associated with known biodegradable bioplastic (polymeric) stents by offering as an alternative a self-expanding braided stent made predominantly of metallic bio-alloys.

The novel hybrid braided stent provides temporary structural support of biological lumens, including but not limited to arteries, cavities, ducts, passages, tracts, and veins.

Magnesium (Mg) is known for its utility in making medical implant devices due to its biocompatibility and degradability, making magnesium an ideal material for clinical use. Mg is also an essential element for the body as it promotes protein synthesis, contributes to nerve and muscle function, bone growth, controls blood sugar, etc. Similar to calcium, potassium, and sodium, Mg is vital to the proper functioning of the human body.

Although Mg and Mg alloys have been used in stents and other types of bioabsorbable stents, several drawbacks exist with conventional methods for producing stents made from Mg and Mg alloys, including lack of radiodensity, unfavorable mechanical properties, and rapid biocorrosion and degradation.

Visibility (i.e., lack of radiodensity) is one of the main drawbacks of many bioabsorbable/degradable implants (FIG. 3C), even though the implant may be visible using alternative imaging modalities, such as intravascular imaging (FIG. 3B). The inability to see a bioabsorbable device under fluoroscopic guidance, for example, can lead to catastrophic adverse events such as clot and scar tissue formation, etc. This in turn can cause diminished blood flow or blockage and, for example, in a heart artery, a myocardial infarct (i.e. a heart attack).

Although a goal of using bioabsorbable/degradable stents is to achieve complete degradation and ultimately resorption of the biomaterial(s) by the body, the present disclosure describes the functionality and utility of hybrid devices in which a combination of absorbable Mg alloys and non-absorbable non-degradable metals are used to produce a self-expanding Mg-based stent which provides radiopacity/radiodensity.

As mentioned above, stent visibility remains a significant impediment of known bioabsorbable/degradable stents/scaffolds. The inability to properly visualize the stent may increase construct failure once implanted in the body, e.g., due to suboptimal or improper placement. The inability to visualize the entirety of the stent construct under, for example, fluoroscopic guidance places both the implant and the patient at risk.

For the former situation (i.e., the risk of product failure), because the user cannot see the implant, they are essentially working blindly. For the latter situation (i.e. the risk to the patient resulting from product failure), the patient is predisposed to potentially severe adverse events, such as those described above. The ability to visualize the bioabsorbable/degradable implant is paramount to the functionality and performance of the device not only during implantation but also in the ensuing period after implantation.

Although difficulties associated with visualizing Mg-based stents under radiation emitting imaging modalities can lead to the stent construct's possible failure, the material from which the stent constructs are made is important. It has been recognized in the scientific literature that current bioabsorbable stent platforms lack sufficient visibility under medical imaging for optimal implantation.

For any functioning stent the manufacturing processes used to fabricate the device should be compatible with the materials so that the underlying properties of the materials are preserved to avoid device performance failures once implanted.

For a bioabsorbable stent made from biometallic materials, manufacturing processes involving focal thermal energy such as welding or laser-cutting or extreme mechanical stress such as wire bending alter the underlying properties of the biometallic materials making them more prone to fracturing or non-uniform biodegradation. Braided manufacturing processes for biometallic materials avoid many of these drawbacks.

Accordingly, disclosed herein are bioabsorbable stents which are improved relative to existing bioabsorbable braided wire stents due to their combination of novel design features which provide radial strength, greater expandability that is more resistant to fracture, and/or enhanced fluoroscopic visibility.

For example, wire-based stents such as those disclosed herein are not affected by heat-affected-zones from a laser which could change the microstructure and properties of the Mg or Mg-alloy. There is also more control over the processing and strength of wires than with extruded tubing: extruded tubing is generally highly annealed to reduce yield strength/recoil and maximize elongation, making it more prone to failure. Hybrid self-expanding bioabsorbable stents (HSEBS) such as those disclosed herein include biodegradable metallic wires and either a degradable magnesium alloy radiopaque composite or non-degradable radiopaque metallic wires made from radiopacifying materials.

The self-expanding feature of the disclosed embodiments arises from a combination of the properties of the bioabsorbable (e.g. Mg or Mg-alloy) wires that make up half or more of the wires of the HSEBS together, and in some cases, with the properties of the non-degradable RO wires, particularly when these wires are braided together and coated with a conformal flexible polymer.

This combination provides a stent which can be compressed prior to delivery in a subject (e.g. a patient) and which, upon release of compression, can self-expand to an expanded position which may be 50% or more of the diameter of the as-made (precompressed) diameter of the HSEBS, which occurs as a result of the combination of the resilience of the wires and the flexible polymer coating as well as the interactions between these components, including the braiding and the adhesion of the coating to the wires and the fact that the coating maintains the interconnections between the wires and the crossover points.

Preferably, the wires of the disclosed embodiments of HSEBS are made in an expanded shape and are then subsequently compressed and constrained in a delivery system. Upon release from the delivery system, the wires "spring back," i.e., self-expand, to a predetermined diameter. The ability of the wires to recover to some or all of the as-made diameter (e.g. recover at least 50% of their as-made diameter) is based at least in part on the elastic properties of the metal(s). In various embodiments, the wires may have a relatively low elastic modulus (e.g. as low as 5 Mpsi/35 GPa) and high yield stress to provide large elastic strains.

In other embodiments, application of a conformal flexible polymer to the braided tube while the tube is in its expanded state provides resilience to the tube so that, when a compressive force on the tube is released, the tube re-expands to some or all of its original pre-compression diameter.

In various embodiments, the HSEBS may be producible from Mg-based wire that either contains rare earth alloying minerals or, in other embodiments, is substantially free of rare earth alloying elements.

The former (i.e., containing rare earth elements) provides an alloy having additional strength, resistance to time-dependent and permanent deformation associated with constant load or stress (i.e., metallic creep), and increased corrosion resistance. Simultaneously, the latter (i.e., free of rare earth elements) eliminates the potential of clinically and histologically induced side effects.

In general, alloys (including Mg alloys) that are free of rare earth minerals and corrosion products are expected to cause fewer to no systematic or local cytotoxicological effects. Thus, the choice of biometal depends on the HSEBS application and/or intended use.

In other embodiments, the HSEBS may also be made from other biodegradable metals alloyed with varying percentages of alloying-metals including alkali and alkaline metals and select transition metals and rare earth elements, including aluminum, calcium, copper, dysprosium, iron, lithium, magnesium, manganese, yttrium, zirconium, and zinc.

Using different biometals and a combination of biometallic alloys can influence the degradation and mechanical behavior of the resulting HSEBS, which, depending on its formulation, may be used in various applications.

In one particular embodiment, Mg may be alloyed (≥90% Mg) with zinc, calcium, and manganese to produce a wire that is strong and ductile, and free of rare earth elements, making the alloy suitable for implantation inside the blood vessels, such as arteries and veins. In one example, the non-rare earth-containing MA includes 1% zinc, 0.3% calcium, and 0.15% manganese with a magnesium content of greater than 98%.

In another embodiment, a Mg-alloy (MA) wire may be provided which includes zinc, zirconium, and rare earth elements and having at least 80% magnesium. This particular MA wire is biocompatible, has high-tensile strength, yield strength, and percent elongation and is composed of 10% dysprosium, 1% neodymium, 1% zinc, and 0.2% zirconium with the balance 87% being magnesium.

As noted above, the radiopacity of stents is important because it allows the device to be visualized during radiographically-guided procedures. The two principal factors that contribute to the radiopacity of a metal are its density and atomic number. Mg allows radiation to pass more freely, making it and its alloys radiolucent because of low density (@ 1.74 g/cm^3) compared to 316L stainless steel (@ 7.99 g/cm^3).

The 316L stainless is mainly made of iron (Fe), has a density of 7.8 g/cm^3, and has an atomic number of 26, compared to the density of Mg which is less than 2 g/cm^3 and has an atomic number of 12. Materials such as Fe that are better at inhibiting the passage of electromagnetic radiation are referred to as radiodense or radiopaque materials.

To enhance the visibility of non-degradable vascular metallic stents, for example, a stent made from 316L stainless steel, in some cases discrete markers (FIG. 5) may be added to the stent (e.g., at the ends) to enhance the visibility of the stent, particularly at its extremities. These markers may be made from gold, platinum, or tantalum. The markers generally have a higher density than the material of the stent, allowing the ends (or other extremities) of the stent to be readily seen angiographically.

However, the significantly lower density of Mg compared to 316L stainless steel makes the Mg-based HSEBS essentially invisible under fluoroscopy. Thus, discrete markers may not by themselves provide sufficient to make an otherwise predominantly radiolucent stent visible under radiography.

Accordingly, instead of using discrete markers, embodiments of the HSEBS devices disclosed herein may include magnesium alloy radiopaque composite or non-degradable radiopaque metallic wires, such as tantalum wire, that spans the entire length of the HSEBS (FIGS. 1, 4 & 6), not just at the extremities (FIG. 5), and thereby confer continuous end-to-end visibility onto the stent. In various embodiments, the non-degradable radiopaque wires may include other biocompatible materials such as gold, platinum, iridium, iron, or tungsten, either alone or as part of an alloy. The addition of radiodense wires thus allows for the stent to be visualized radiographically, allowing an otherwise transparent biometallic wire stent to be visible under fluoroscopic guidance (FIG. 4).

Various numbers of radiopaque wires may be used. Certain embodiments of the disclosed HSEBS device may include at least two radiopaque (RO) wires, while in other embodiments no more than four RO wires may be woven into the structure of the HSEBS (FIGS. 2 & 4). In certain embodiments the RO wires extend along the full length of the braided construct (FIG. 6). The RO wires may be disposed opposite (or 180° apart) from one another when viewed in cross-section in the two and four-RO-wire configurations (101 and 102), whereas the wires may be placed 90° apart for the four-wire configuration for optimal visibility and for added design stability (103). More generally, in various embodiments the RO wires may be spaced approximately equally apart from one another when viewed in cross-section, although other distributions are also possible.

In some embodiments the RO wires may be absorbable and may include a radiopaque material for example as a core. In particular embodiments, a magnesium composite wire having a radiopaque core may be used. For example, a drawn filled tube (DFT^{®}) wire (Fort Wayne Metals, IN) having an extruded material shell of one element or alloy (e.g. Mg or nitinol) and a core including another metallic/non-metallic material is an established material for use in the production of medical devices and may be used as the RO wire.

In certain embodiments, MA tubes (e.g., a drawn filled Mg tube) may be used which are filled with powdered radiopaque materials to produce a RO composite wire having a biodegradable shell that provides both the mechanical properties that are more similar to those of non-absorbable metals, which can make the stent or other device which includes the MA tubes both degradable and radiopaque. The radiopacifying powder may include one or more known radiopacifiers, including barium, bismuth, tantalum, and tungsten, and may make up to 40% of the area of the core material within the magnesium alloy composite wire.

In particular embodiments, the powdered radiopaque material that fills the MA tubing may preferably be non-metallic radiopaque materials such as those which are often used in medicine, such as bismuth trioxide (Bi₂O₃). Other fillers (or radiopacifiers) are typically made of dense powders, including barium compounds (e.g., barium sulfate (BaSO₄), tantalum oxide (Ta₂O₅), or tungsten carbide (WC)), and may have a sufficient effect on energy attenuation of x-ray beams as the beams penetrate the material, so as to reduce the beam's intensity by absorbing or deflecting all or a portion of the beam.

The hollow MA alloy tubing may be produced using contemporary work-hardening (cold-work) methods. For use in stent manufacturing, the outer diameter of the hollow MA tube may range from 0.1 mm to 1 mm, while the RO materials may make up between 10% and 40% of the area of the inner core material.

A composite RO wire is an alternative to non-degradable RO wire in certain applications, such as vascular stenting where leaving nothing behind in the body has its advantages, particularly in neurovascular and coronary stenting applications, where the vessels are smaller and prone to re-occlusion, as well as in some non-vascular/venous applications, such urethral stenting and bile duct stenting where fluoroscopic guidance may be needed to visualize the stent and where the stent degrades or disappears over time. This is particularly useful where stent removal is indicated (e.g., in ureteral stenting).

In other embodiments, the non-degradable RO wires may be made from metals having elastic properties with lower elastic modulus and high yield stress for large elastic strains and thus may serve as the stent's non-resorbable backbone, such as cobalt-alloys and nitinol (FIG. 2; 102). Accordingly, in various disclosed embodiments the non-degradable RO wire may serve both to provide a radiopacity role and to provide certain mechanical properties in the braided structure, for example where the non-degradable RO wires are placed in a "crisscrossed" fashion.

In various embodiments the non-degradable RO wires may also be made from a shape memory alloy, such as nickel-titanium (NiTi or nitinol). In this case, a more significant strain may be achievable either due to the alloy's super-elasticity and/or by leveraging the material's thermal memory. Nitinol wire may be used to provide additional mechanical support to the braided construct.

In certain embodiments the nitinol wire may also contain a core wire to enhance visibility by creating a nitinol composite. In one particular embodiment, NiTi DFT^{®} wires (Fort Wayne Metals, IN) may be used which combine radiodense materials, including, but not limited to, the most commonly used RO marker materials, such as gold, platinum, tantalum, and radiodense powders/pacifiers as previously described, with the result that the other RO materials may make up between 10% and 40% of the core material. In this case, the nitinol composite provides both visibility and mechanical support to the braided structure and thus may help provide self-expansion of the HSEBS.

The non-degradable metallic materials that may make up the construct of various embodiments of the HSEBS are dissimilar to one another chemically and could lead to biometallic corrosion or galvanic corrosion. Galvanic corrosion takes place when two different metals are in contact in the presence of an electrolyte. Electrolytes are found in all bodily fluids, including blood, and may include sodium, calcium, potassium, and magnesium salts. Galvanic corrosion occurs with the corrosion taking place at and near the point where the two metals contact. The HSEBS has a plurality of these contact points.

Accordingly, to reduce or eliminate the galvanic effect of the dissimilar metals that can make up the HSEBS construct, a dielectric, insulating permanent (i.e. non-biodegradable) polymer coating such as polyimide (PI) may be applied to the RO wire. PI, an imide monomer, may be applied to insulate the non-degradable metallic wires.

In various embodiments, the polymer may be made from a dielectric, insulating material that can withstand very high temperatures, including polyimide and fluoropolymers (e.g., Moldflon PTFE and PFA). These polymers can be selected based on the metals used, the application and location where the device will be used, and how the materials are ultimately processed, e.g., braided, annealed, and/or heat treated.

To prevent rapid degradation of the biometallic, Mg-based substrate, biodegradable polymers may be utilized. The use of certain biodegradable polymer coatings to modulate and slow Mg-based stents degradation is known to those skilled in the art and reported in the scientific literature.

In various embodiments, a conformal biodegradable polymer (CBP) may be applied to the HSEBS to bind the wires at the crossover point to prevent them from sliding. The CBP coating adheres to the surface of the stents. The CBP coating may also prevent wire ends from deformation and may allow the braided stent to achieve uniform expansion once it is unconstrained (FIG. 10). The left image in FIG. 10, which shows a stent coated with a CBP that does not include a plasticizer, includes a dashed line which shows the original location of a wire which has detached at several crossover points and as a result has slid relative to the other wires (displacement indicated by the arrows). On the other hand, the wires of the stent in the right image in FIG. 10, which is coated with a CBP which includes a plasticizer, have not detached at crossover points. Thus, the plasticizer helps prevent deformation of the wires and helps prevent the wires from detaching at the crossover points, particularly in the open-ended configured stents such as those shown in FIG. 10.

In some embodiments, conformal biodegradable polymers suitable for this application include but are not limited to: poly(L-lactide) or PLLA; poly (DL-Lactide) or PLA; Poly(L-lactide-co-D, L-lactide); or polyglycolide or PGA; poly(caprolactone) as well as their copolymers such as poly(lactide-co-glycolide). In various embodiments, the biodegradable polymers may also include drug-eluting components such as those disclosed in U.S. Patent No. 9.849,008, which is incorporated by reference herein in its entirety. In some embodiments, the biodegradable polymer may contain or may have coated thereon an antiproliferative drug-containing coating which may include a limus-based (i.e. rapamycin or its derivatives) drug or a taxane-based drug.

In certain embodiments, the conforming properties of the polymer may optionally be modified with the use of a biocompatible plasticizer. Suitable plasticizers include but are not limited to: alkyl citrates, such as triethyl citrate, acetyl triethyl citrate, tributyl citrate, acetyl tributyl citrate, or tri-(2ethylhexyl)citrate.

In particular embodiments, the conformal biodegradable polymer may include poly (D, L-lactide-co-glycolide) and acetyl tri-n-butyl citrate (ATBC) plasticizer. In some embodiments, the polymer with plasticizer may help in the elastic recovery of the stent during deployment. The addition of the plasticizer to the biodegradable polymer gives the conformal coating more elasticity, reducing the risk of coating delamination and tearing during cycling of the stent/PPS. Moreover, the biodegradable polymer plasticizer helps with the elastic recovery of the stent to a recovered state from a constrained state.

The present disclosure provides sufficient detail to allow someone skilled in the art of braiding implantable medical devices to produce one or more embodiments of the disclosed device (HSEBS), for example using commercially available and programmable braiders, with the desired wire count, braid pattern, and angle.

In one embodiment, a HSEBS may include biodegradable metallic wires, such as magnesium-alloy radiopaque composite wire or non-degradable RO wires, such as Ta, made from transition metals, which are dielectrically-coated with a permanent coating, such as with PI, providing a device having enhanced visibility and which is covered by a conformal biodegradable polymer coating, such as PLGA.

In a preferred embodiment an automated braiding system having programmable pick counts, braid lengths, and braiding speed may be used, enabling the production of braided designs that, for example, have more flexibility, stiffness, kink resistance, better torque response, and radial/hoop strength.

A preferred braided construct utilizes carriers designed to unroll wire from horizontally placed bobbins that enable braiding of the most tension-sensitive materials without rolling or twisting. However, other braiding/weaving methods can be used to achieve the desired braided tube construct.

Preferably, the HSEBS are interlaced in a one-over, one-under (1x1) pattern that first produces a braided tube by winding a plurality of both biodegradable and non-degradable wires in a clockwise and counterclockwise manner around a single mandrel. Nevertheless, in various embodiments other patterns may be used for the braided tube.

Mandrel size, wire-count per braided tube construct, wire diameter and properties, and braid angle are also factors that affect braid performance, such as elastic recovery, hoop, and/or radial strength, producing a preferred braided tube construct in a continuous length on a single mandrel, in a 1x1 pattern.

In a preferred embodiment the braided tube construct may be made of 24 wires. Twenty-four wire tubular structure maintains the construct stability for a preferred wire and braiding mandrel size and target braid angle. The 24-wire braided tube construct for a preferred HSEBS embodiment minimizes deformation of the Mg wires at intersecting points.

In a preferred embodiment, the bioabsorbable magnesium (Mg) wire is alloyed with materials either containing or free of rare earth elements, having an outer diameter ranging from 0.1 mm to 0.2 mm.

For example, the Mg-wire containing rare earth elements may include weight concentrations (%w/w) ranging from 80-90% magnesium and be alloyed with rare earth materials such as greater than 10 %w/w of dysprosium to give the Mg-wire higher strength and may contain other alloying elements, such as neodymium, zinc, and zirconium with a combine %w/w ranging from 0.1-5%. Another example is a Mg-wire free of rare-earth materials comprising greater than 95% magnesium and other alloying elements, such as zinc, manganese, and zirconium ranging in weight concentration from 0.1% to 5%.

The braided tube construct has a braid angle of at least 50° measured along the longitudinal axis of the mandrel/stent at the point where the wires intersect, i.e., the braid angle is the angle between an intersecting wire and a line parallel the long axis of the tube (see angle α in Fig. 6). In certain preferred embodiments, the braid angle is between 60° and 65°.

The biodegradable wire of the braided construct may include a biodegradable magnesium-alloy radiopaque composite wire having an outer diameter similar to that of the non-composite magnesium wire (e.g., ranging from 0.1 mm to 0.2 mm,) where the RO core of the magnesium-alloy radiopaque composite wire makes up 10-40% of the wire's diameter.

In comparison, the diameter of the non-degradable RO wire when included in the braided tube is generally smaller than that of the biodegradable wire.

In various embodiments, the non-degradable RO wire diameter may be between 25% and 40% smaller than that of the diameter of the magnesium alloy wire, in part to compensate for the mechanical properties of the non-degradable RO wire and in part to avoid distorting the braided tube.

In some embodiments, tantalum (Ta) wire may be used as the non-degradable RO wire to enhance the visibility or radiopacity of the HSEBS, allowing the generally radio-transparent biometallic braided stent to be visible under fluoroscopic guidance. However, other materials that are both considered biocompatible and radiodense, including gold, iridium, iron-alloys, platinum, platinum-group elements, silver, titanium, tantalum, tungsten can be used.

The Ta wire is generally stronger than the biodegradable Mg wire used in producing the braided tube construct of preferred embodiments of the HSEBS (~250ksi for the Ta wire compared to ~60ksi UTS for the Mg-alloy biodegradable wire). The modulus for Ta is higher (~21 Mpsi vs. ~5 Mpsi), so at the given diameters (e.g., 100 µm for Ta and 150 µm for Mg), the flexural stiffness is roughly equivalent.

The ratio of RO wires to biodegradable wires for a preferred embodiment should not exceed 1:11 in the 24-wire configuration as this means that most (>90%) of the braided tube is to be bioabsorbable unless a composite non-degradable wire is used, in which case a wire ratio of 1:5 may be used for the 24-wire braided construct.

In a preferred embodiment, the non-degradable RO wire component of the HSEBS is coated with a dielectric, insulating permanent polymer coating, such as polyimide (PI), to insulate non-degradable metallic wires to thereby prevent galvanic corrosion between the dissimilar metals that make up the braided stent.

PI is used in a preferred embodiment because the PI monomer provides excellent dielectric insulative and mechanical properties as well as chemical inertness. Compared to other fluoropolymers, PI can be applied in multiple thin layers to improve the substrate's bond. PI can also withstand very high temperatures with a continuous use maximum of 240°C (464°F) and resistance to short exposures up to 400°C (752°F), with minimal generation of volatiles.

The hybrid braided tube construct may be cut into shorter segments to produce the HSEBS. For stability, the braided tubes are generally cut into stents at a point where the wires complete at least one full revolution (360°) along the long axis on the mandrel to reduce the likelihood of the wire ends unraveling.

Preferably, the hybrid tube undergoes a first cut to produce a shorter braided segment from the longer hybrid tube followed by a finishing-cut to produce an uniform end length to the stent to reduce the risk of wire deformation and improve stent symmetry and uniformity.

In a preferred embodiment the biodegradable polymer includes poly(D, L-lactide-co-glycolide) and acetyl tri-n-butyl citrate (ATBC) plasticizer. As previously described, the CBP coating prevents wire deformation and shifting and also provides resilience which allows the braided stent to achieve uniform expansion once unconstrained. It also helps in the elastic recovery of the stent during stent deployment.

A preferred polymer is Poly (D, L-lactide-co-glycolide) Resomer^{®} RG 858 S (Merck KGaA, Darmstadt, Germany), which is a softer, amorphous material having a relatively fast degradation rate (e.g. < 9 months). The ATBC plasticizer is a low volatility compound with a good plasticization effect. It is a safe plasticizer utilized across many industries, including the production of medical products.

In various embodiments, the CBP coating, with or without plasticizer, may be applied evenly to some or all of the stent, resulting in a coating with an approximately uniform thickness of at least 5 micrometers covering the stent. However, in various embodiments the CBP coating thickness may be applied at thicknesses of less than and/or greater than 10 micrometers to speed up corrosion or, conversely, extend the longevity of the mechanical properties of the biodegradable metal. The CBP coating can be applied by spraying layers and/or dip-coating the braided stent, creating a conformable coating.

Additionally, the CBP coating can be applied in a thicker layer at the edges or ends of each stent segment, with or without a plasticizer, in order to form discrete polymer rings (FIG. 11) to thoroughly encapsulate the open-ended wire ends. Producing the polymer rings at the ends of the stent segments can be achieved by a number of procedures including dipping, spraying, and/or painting the rings onto the edges of the stents one or more times in the polymer solution and/or by applying the polymer to the stent's edges using a higher concentration of polymer solution (e.g., 2x).

The polymer rings protect the stents, and particularly the ends of the stents, by encapsulating the wire ends of the braided stent from wire-end-deformation during the stent's loading and deployment. The thicker layer may be applied at both ends (or edges) of the HSEBS of the stent, which in various embodiments may cover between 5% and 40% of the entire stent length at each edge, and preferably between 10 and 20% of the whole stent length at each edge. In other embodiments discrete polymer rings - up to 50% of the stent - may be applied to fix/suspend the braided hybrid stent's wire ends to prevent wire-end bending and distortion (FIG 11).

In still other embodiments, the disclosed biodegradable stent may be made only using biodegradable wires (i.e., without RO wires braided into the stent) where non-degradable metallic bands (FIGS. 8 and 9) may be used to join pairs of wire ends to create closed ends, which provides structural stability and also provides radiopacity to the stent. Methods for joining pairs of wire ends are known to those skilled in the art. In one embodiment, pairs of wires may be joined by, e.g., insulated, radio-dense joining cuffs, e.g., as described in WO 2018/145029 A1, incorporated herein by reference in its entirety.

In other embodiments, the HSEBS may be entirely covered/coated in a higher viscosity CBP coating to create a graft-like covering that envelops the entire stent. The term "viscous" coating, in this case, refers to a coating with elastomeric properties, having a relatively low Young's modulus that covers the whole braided tube and the areas/cells between the wires at the crossover points to create a sleeve or graft-like covering (FIG. 11).

These sleeve-like coatings are composed of at least one biodegradable polymer or biodegradable copolymer or mixture thereof. In a preferred embodiment the biodegradable polymer is a polylactic acid co glycolic acid copolymer (PLGA) of relatively high inherent viscosity between 0.5 and 2.5 dl/g and preferably between 1 and 2 dl/g.

The coating can optionally contain at least one additive that is used to enhance the elastomeric properties of the coating with additives having a mass ratio of 1% to 50% of the coating weight and preferably between 5% and 15% of the coating weight.

In some embodiments the additive may be a bio-based plasticizer, meaning a plasticizer that is biodegradable and/or can be metabolized by the body cells. In yet other embodiments the additive is selected from the family of alkyl citrates including but not limited acetyl triethyl citrate, tributyl citrate, acetyl tributyl citrate, tri-(2ethylhexyl)citrate, acetyl trioctyl citrate, trihexyl citrate.

Coatings of this type can be applied by a variety of methods including dipping, spraying, and brushing. Preferably the sleeve-like coating is applied to the stent with an automated dipping method using concentrated solutions of the coating materials in appropriate solvents, such as chloroform, dichloromethane, dimethyl sulfoxide. The sleeve-like coating can be achieved by a dipping method with the use of solutions containing between 2 and 12% (i.e., a higher viscosity) by weight of the polymer, the balance of the weight being solvent, preferably between 4 and 6% by weight.

In various embodiments, the braided pattern may be 2x2 (two-over, two-under) (FIG. 8), and in various other embodiments other suitable braiding patterns may be used. Furthermore, in certain embodiments the braided tube may be made on any standard braiding machine, using either a horizontal or vertical braider.

The braided construct can also be produced generally using any multiple of 4 wires, with the most common configurations being 8, 12, 16, 24, 32, 48, 64, 72, 96, 144, and up to 288 wires using various commercially-available braiders. In general, half of the total number of wires will be wound in one direction with the other half being wound in the opposite direction, producing a crossing braided pattern. In one particular embodiment a preferred number of wires is twenty-four (24), where the 24-wire tubular structure maintains the stability of the HSEBS for a preferred wire and braiding mandrel size and target braid angle and minimizes the deformation of the wires at intersecting points.

For other applications and/or embodiments, the outer diameter of the wire may range from 0.1 mm to 0.2 mm; however, in various embodiments the outer diameter may range from 0.01 mm to 1.0 mm, depending on the type of biodegradable metal, the target braid angle, and the number of wires needed to produce the braided tube. Furthermore, other factors such as the size of the stent or other implantable device and its intended target (e.g. in a large vessel or smaller artery) will also help determine the diameter of the wire that is used, with larger implantable structures using larger-diameter wires and smaller structures using smaller-diameter wires.

In various embodiments, the size/diameter of the wire and the mandrel, as well as the number of wires per braided tube, may affect the braid angle, but in general the angle should not be less than 30°. In some embodiments, the braid angle may be between 60° and 65° for a braided construct having 24 wires. In particular embodiments, the braid angle for braided constructs having ≥32 wires may be at least 50°.

In various embodiments, the RO wire may be used to enhance the visibility of the HSEBS, allowing the mainly transparent biometallic braided stent to be visible under fluoroscopic guidance. As described, other biocompatible materials and radiopacifying powders may be used to produce either magnesium-alloy radiopaque composite or non-degradable radiodense wires.

In particular embodiments, the ratio and/or arrangement of permanently coated non-degradable metallic wires to biodegradable biometallic wires may be no more than 1:1 of the total number of wires needed to produce the braided tube. In certain embodiments, the ratio of non-degradable to degradable wires can be as low as 1:1 or as high as 1:5, 1:11, 1:71, or other intermediate ratios. In one particular embodiment for a 24-wire braided configuration, the ratio may not exceed 1:11, as most (>90%) of the braided construct is to be absorbable unless composite wires are used. In other embodiments the ratio of composite wires to biodegradable metallic wires may be 1:5 in a 24-wire braided construct and no less than 1:71 for a 288-wire braid.

In other embodiments, the hybrid braided tube may undergo the same cutting process as a preferred embodiment (i.e., a first rough cut followed by a second cut to trim the wire ends) as described.

The hybrid tube may also experience the first cut to detach a shorter segment of the hybrid tube and a second cut where the wires complete an additional one-quarter revolution along the mandrel's long-axis (element (a) in FIGS. 7A & 7B). Then, pairs of wire-ends may be separated and aligned parallel to one another and secured with a polymer cuff (FIGS. 7A & 8). Once each pair of end-wires is connected with cuff 20, the wire-ends protruding from the cuffed ends may be trimmed (FIG 7A) to form a closed-end stent.

Alternatively, the hybrid tube may also experience the first cut to detach a shorter segment of the hybrid tube and a second cut where the wires complete an additional one-quarter revolution along the mandrel's long-axis (element (a) in FIGS. 7A & 7B). Then, pairs of wire-ends may be aligned parallel to one another to form a loop that is approximately perpendicular to the longitudinal axis of the stent and secured with a polymer cuff 20 (FIGS. 7B & 9).

Thus, while the invention has been described above in connection with particular embodiments and examples, the invention is not necessarily so limited, and that numerous other embodiments, examples, uses, modifications and departures from the embodiments, examples and uses are intended to be encompassed by the claims attached hereto.
The following clauses, which are not claims, relate to various aspects and embodiments of the invention:
1. An implantable device, comprising:
   a tube comprising a plurality of biodegradable biometallic wires braided together,
   the tube being coated with a flexible conformal biodegradable polymer in an expanded state such that, upon compression and release of compression, the flexible conformal biodegradable polymer-coated tube self-expands back to the expanded state.
2. The implantable device of clause 1, wherein the tube further comprises one or more radiopaque wires to provide radiopacity to the tube.
3. The implantable device of clause 2, wherein at least one of the one or more radiopaque wires comprises a composite wire.
4. The implantable device of clause 3, wherein at least one of the one or more radiopaque wires comprises a magnesium-alloy radiopaque composite wire.
5. The implantable device of clause 4, wherein a diameter of the magnesium-alloy radiopaque composite wire is substantially the same as a diameter of each of the plurality of biodegradable biometallic wires.
6. The implantable device of clause 5, wherein the magnesium-alloy radiopaque composite wire comprises a radiopacifying filler.
7. The implantable device of clause 6, wherein the radiopacifying filler includes at least one of barium, bismuth, tantalum, or tungsten.
8. The implantable device of clause 7, wherein the magnesium-alloy radiopaque composite wire comprises a shell surrounding a core, and
   wherein the core comprises the radiopacifying filler and has a higher density than the shell.
9. The implantable device of clause 3, wherein the one or more radiopaque composite wires comprises a shape memory alloy.
10. The implantable device of clause 9, where the shape memory alloy comprises a nickel-titanium alloy.
11. The implantable device of clause 2, wherein the one or more radiopaque wires comprises one or more non-degradable radiopaque wires.
12. The implantable device of clause 11, wherein the one or more non-degradable radiopaque wires comprises a biocompatible radiopaque metal.
13. The implantable device of clause 12, wherein a diameter of the one or more non-degradable radiopaque wires is between 25% and 40% smaller than a diameter of each of the plurality of biodegradable biometallic wires.
14. The implantable device of clause 13, wherein the biocompatible radiopaque metal includes at least one of gold, platinum, tantalum, iridium, iron, or tungsten.
15. The implantable device of clause 14, wherein the one or more non-degradable radiopaque wires is coated with a permanent polymer coating to inhibit galvanic corrosion.
16. The implantable device of clause 15, wherein the permanent polymer coating comprises a dielectric insulating material.
17. The implantable device of clause 16, wherein the permanent polymer coating comprises at least one of polyimide or a fluoropolymer.
18. The implantable device of clause 1, wherein the plurality of biodegradable biometallic wires comprises non-composite magnesium wires.
19. The implantable device of clause 1, wherein the plurality of biodegradable biometallic wires comprises a composite wire.
20. The implantable device of any one of clauses 1 to 19, wherein the plurality of biodegradable biometallic wires is substantially free of rare earth elements.
21. The implantable device of any one of clauses 1 to 19, wherein the plurality of biodegradable biometallic wires comprises at least 80% w/w magnesium.
22. The implantable device of clause 21, wherein the plurality of biodegradable biometallic wires further comprises at least one of zinc or zirconium.
23. The implantable device of clause 22, wherein the plurality of biodegradable biometallic wires further comprises at least one rare earth element.
24. The implantable device of any one of clauses 1 to 19, wherein the plurality of biodegradable biometallic wires comprises a magnesium alloy including at least 90% w/w magnesium and at least one of zinc, calcium, or manganese.
25. The implantable device of any one of clauses 1 to 19, wherein the plurality of biodegradable biometallic wires further comprises at least one of an alkali metal, an alkaline metal, a transition metal, or a rare earth metal.
26. The implantable device of any one of clauses 1 to 19, wherein the plurality of biodegradable biometallic wires further comprises at least one of aluminum, calcium, copper, dysprosium, lithium, and magnesium, manganese, yttrium, or zirconium.
27. The implantable device of any one of clauses 1 to 19, wherein a first portion of the plurality of biometallic wires is wound in a clockwise direction and a second portion of the plurality of biometallic wires is wound in a counterclockwise direction.
28. The implantable device of clause 27, wherein the first portion of wires is braided with the second portion of wires in a one-over, one-under (1x1) pattern.
29. The implantable device of clause 27, wherein the first portion of wires is braided with the second portion of wires in a two-over, two-under (2x2) pattern.
30. The implantable device of any one of clauses 1 to 19, wherein a diameter of each of the plurality of biodegradable wires is between 0.01 mm and 1.0 mm.
31. The implantable device of clause 30, wherein the diameter of each of the plurality of biodegradable biometallic wires is between 0.1 mm and 0.2 mm.
32. The implantable device of clause 2, wherein a total number of the plurality of biodegradable biometallic wires and the one or more radiopaque wires in the tube comprises a multiple of four wires.
33. The implantable device of clause 32, wherein the total number of the plurality of biodegradable biometallic wires and the one or more radiopaque wires in the tube is 4, 8, 12, 16, 24, 32, 48, 64, 72, 96, 144, or 288 wires.
34. The implantable device of clause 33, wherein the total number of the plurality of biodegradable biometallic wires and the one or more radiopaque wires in the tube is 24.
35. The implantable device of clause 33, wherein each of the plurality of biodegradable wires is wound at an angle of at least 30° relative to a longitudinal axis of the tube.
36. The implantable device of clause 33, wherein, when the tube has 24 wires, the angle is between 60° and 65°.
37. The implantable device of clause 33, wherein, when the tube has at least 32 wires, the angle is at least 50°.
38. The implantable device of clause 2, wherein a ratio of the one or more radiopaque wires to the plurality of biodegradable biometallic wires in the tube is no more than 1:1.
39. The implantable device of clause 2, wherein, when the tube has 24 wires, the ratio of the one or more radiopaque wires to the plurality of biodegradable biometallic wires in the tube is no more than 1:11.
40. The implantable device of clause 2, wherein, when the tube has 24 wires, the ratio of the one or more radiopaque wires to the plurality of biodegradable biometallic wires in the tube is no more than 1:5.
41. The implantable device of clause 2, wherein, when the tube has 288 wires, the ratio of the one or more radiopaque wires to the plurality of biodegradable biometallic wires in the tube is no more than 1:71.
42. The implantable device of any one of clauses 1 to 19, wherein the tube is cut into at least two segments.
43. The implantable device of clause 42, wherein, in each of the at least two segments of the tube, each of the wires completes at least one full revolution around a long axis of the tube.
44. The implantable device of clause 43, wherein each end of each of the at least two segments undergoes a finishing-cut to produce substantially uniform lengths of wire ends.
45. The implantable device of clause 1, wherein, upon the release of compression, the flexible polymer-coated tube self-expands to at least 50% of the expanded state.
46. The implantable device of any one of clauses 1 to 19, wherein the flexible polymer that coats the tube comprises a biodegradable polymer.
47. The implantable device of clause 46, wherein the flexible polymer comprises a conformal coating.
48. The implantable device of clause 47, wherein the flexible polymer binds the wires of the tube together at a plurality of wire crossover points to inhibit sliding movement between the wires.
49. The implantable device of clause 48, wherein the flexible polymer is applied to the tube by at least one of spray-coating or dip-coating to produce at least one of a conformal coating or sleeve-like coating.
50. The implantable device of clause 47, wherein the biodegradable polymer further comprises at least one of: poly(L-lactide) (PLLA); poly (DL-Lactide) (PLA); poly(L-lactide-co-D, L-lactide); polyglycolide (PGA); poly(D, L-lactide-co-glycolide); or poly(caprolactone).
51. The implantable device of clause 47, wherein the biodegradable polymer further comprises at least one alkyl citrate biocompatible plasticizer including triethyl citrate, acetyl triethyl citrate, tributyl citrate, acetyl tributyl citrate, or tri-(2ethylhexyl)citrate.
52. The implantable device of clause 47, wherein the biodegradable polymer comprises a conformal polymer comprising poly (D, L-lactide-co-glycolide) and acetyl tri-n-butyl citrate (ATBC) plasticizer.
53. The implantable device of clause 46, wherein the biodegradable polymer further comprises an antiproliferative drug or an antiproliferative drug-containing coating.
54. The biodegradable polymer of clause 53, wherein the polymer or the antiproliferative drug-containing coating comprises a limus-based drug or a taxane-based drug.
55. The implantable device of any one of clauses 1 to 19, wherein the flexible polymer is applied to the tube at a thickness of at least 5 µm.
56. The implantable device of clause 55, wherein the flexible polymer is applied to the tube at a thickness of greater than 5 µm at each end of the tube.
57. The implantable device of clause 55, wherein the flexible polymer is applied to each end of the tube at a thickness of at least 20 µm.
58. The implantable device of clause 55, wherein each end of the tube having the flexible polymer applied at a thickness of at least 20 µm covers between 10% and 20% of a length of the tube.
59. The implantable device of clause 55, wherein the flexible polymer at each end of the tube comprises a thickened band which encapsulates wire ends of the tube.
60. The implantable device of clause 55, wherein a sleeve-like cover is created by dipping the stent into a solution containing between 2% and 12% by weight of the polymer, the balance of the weight being solvent between 4% and 6% by weight.
61. The implantable device of any one of clauses 1 to 19, wherein ends of the plurality of biodegradable biometallic wires are joined together to form a closed-ended stent.
62. The implantable device of any one of clauses 1 to 19, wherein ends of the plurality of biodegradable biometallic wires are not joined together to form an open-ended stent.

## Claims

1. An implantable device, comprising:
a tube comprising a plurality of biodegradable biometallic wires braided together,
the tube being coated with a flexible conformal biodegradable polymer in an expanded state such that, upon compression and release of compression, the flexible conformal biodegradable polymer-coated tube self-expands back to the expanded state,
wherein the flexible conformal biodegradable polymer binds the braided wires of the tube together at a plurality of wire crossover points to inhibit sliding movement between the wires, and
wherein the flexible conformal biodegradable polymer comprises a plasticizer.

2. The implantable device of claim 1, wherein the tube further comprises one or more radiopaque wires to provide radiopacity to the tube.

3. The implantable device of claim 2, wherein at least one of the one or more radiopaque wires comprises a composite wire.

4. The implantable device of claim 3, wherein at least one of the one or more radiopaque wires comprises a magnesium-alloy radiopaque composite wire.

5. The implantable device of claim 1, wherein the one or more radiopaque wires comprises one or more non-degradable radiopaque wires.

6. The implantable device of claim 5, wherein the one or more non-degradable radiopaque wires comprises a biocompatible radiopaque metal.

7. The implantable device of claim 6, wherein a diameter of the one or more non-degradable radiopaque wires is between 25% and 40% smaller than a diameter of each of the plurality of biodegradable biometallic wires.

8. The implantable device of claim 7, wherein the biocompatible radiopaque metal includes at least one of gold, platinum, tantalum, iridium, iron, or tungsten.

9. The implantable device of claim 8, wherein the one or more non-degradable radiopaque wires is coated with a permanent polymer coating to inhibit galvanic corrosion.

10. The implantable device of claim 9, wherein the permanent polymer coating comprises a dielectric insulating material.

11. The implantable device of claim 10, wherein the permanent polymer coating comprises at least one of polyimide or a fluoropolymer.

12. The implantable device of any preceding claim, wherein the plurality of biodegradable biometallic wires comprises at least 80% w/w magnesium; or
wherein the plurality of biodegradable biometallic wires comprises a magnesium alloy including at least 90% w/w magnesium and at least one of zinc, calcium, or manganese; or
wherein the plurality of biodegradable biometallic wires further comprises at least one of an alkali metal, an alkaline metal, a transition metal, or a rare earth metal.

13. The implantable device of any one of claims 1 to 11, wherein the tube is cut into at least two segments, and
wherein, in each of the at least two segments of the tube, each of the wires completes at least one full revolution around a long axis of the tube.

14. The implantable device of any one of claims 1 to 11, wherein the flexible biodegradable polymer comprises a conformal coating.

15. The implantable device of claim 14, wherein the flexible biodegradable polymer further comprises at least one of: poly(L-lactide) (PLLA); poly (DL-Lactide) (PLA); poly(L-lactide-coD, L-lactide); polyglycolide (PGA); poly(D, L-lactide-co-glycolide); or poly(caprolactone); or
wherein the flexible biodegradable polymer further comprises at least one alkyl citrate biocompatible plasticizer including triethyl citrate, acetyl triethyl citrate, tributyl citrate, acetyl tributyl citrate, or tri-(2ethylhexyl)citrate; or
wherein the flexible biodegradable polymer comprises a conformal polymer comprising poly (D, L-lactide-co-glycolide) and acetyl tri-n-butyl citrate (ATBC) plasticizer; or
wherein the flexible biodegradable polymer further comprises an antiproliferative drug or an antiproliferative drug-containing coating.
